Europäisches Patentamt

European Patent Office

Office européen des brevets

(19)

(11) Veröffentlichungsnummer: **0 235 606**
**B1**

(12)

# EUROPÄISCHE PATENTSCHRIFT

(45) Veröffentlichungstag der Patentschrift:
**13.06.90**

(51) Int. Cl.⁵: **A61F 2/34**

(21) Anmeldenummer: **87101571.5**

(22) Anmeldetag: **05.02.87**

(54) In einem Knochenzementbett zu verankernde Endoprothese für eine Hüftgelenkspfanne.

(30) Priorität: **18.02.86  CH 652/86**

(43) Veröffentlichungstag der Anmeldung:
**09.09.87 Patentblatt 87/37**

(45) Bekanntmachung des Hinweises auf die Patenterteilung:
**13.06.90 Patentblatt 90/24**

(84) Benannte Vertragsstaaten:
**AT DE FR GB IT**

(56) Entgegenhaltungen:
**EP-A- 0 153 523**
**DE-U- 8 623 855**
**FR-A- 2 215 927**
**US-A- 4 566 138**

(73) Patentinhaber: **GEBRÜDER SULZER
AKTIENGESELLSCHAFT, Zürcherstrasse 9,
CH-8401 Winterthur(CH)**
Patentinhaber: **Protek AG, Stadtbachstrasse 64,
CH-3001 Bern(CH)**

(72) Erfinder: **Müller, Maurice E., Prof.Dr.-med.,
Melchenbühlweg 9, CH-3006 Bern(CH)**

(74) Vertreter: **Sparing Röhl Henseler Patentanwälte
European Patent Attorneys, Rethelstrasse 123,
D-4000 Düsseldorf 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft eine in einem Knochenzementbett zu verankernde Endoprothese für eine Hüftgelenkspfanne, bestehend aus einem inneren Kunststoff-Pfannenkörper, der die eigentliche Pfannenschale aufnimmt, und aus einem mehrlagigen Metallgitter, das auf der im wesentlichen halbkugelförmigen äusseren Oberfläche des Pfannenkörpers fixiert ist.

Zur Versteifung von Endoprothesen aus Kunststoff für Hüftgelenkspfannen ist bereits vorgeschlagen worden, die Aussenfläche des Pfannenkörpers mit einem mehrlagigen Metallgitter oder Drahtnetz zu "beschichten", wobei eine Lage zur innigen Verbindung von Kunststoff und Metall in den Kunststoff eingebettet ist und die restlichen Lagen zur Aufnahme einwachsenden Knochengewebes bestimmt sind.

Werden diese zwar mit der Feinstruktur des Gitters versehenen, im übrigen jedoch strukturlosen Pfannen in einem Knochenzementbett verwendet, so dringt der bei der Implantation noch nicht ausgehärtete Knochenzement einerseits in die "freien Poren" des Metallgitters und andererseits - aufgrund des beim Einpressen der Pfanne ausgeübten Druckes - in das spongiose Knochengewebe des Beckenknochens ein. Da es sich bei Knochenzement nicht um einen Klebstoff mit Adhäsionswirkung handelt, sondern die verankernden Kräfte allein durch ein mechanisches "Verhaken" des Zements mit dem Netz des Implantats bzw in den Poren des Knochengewebes erzeugt werden, hat es sich in der Praxis gezeigt, dass die Verankerung ungenügend ist, wenn die vom Knochenzement durchsetzten "Schichten" der Spongiosa und des Metallgitters unmittelbar aufeinander aufliegen; für eine gute Haftung muss vielmehr mindestens auf Teilen der "Aussenhaut" der Pfanne eine gewisse Mindesttiefe an Knochenzementbett vorhanden sein. Bei den geschilderten, abgesehen von der Gitterstruktur des Metallgitters strukturlosen Pfannen ist diese Mindestdicke eines Zementbettes zwischen Implantat und Knochen nicht mehr gewährleistet, da der Knochenzement beim Einpressen der Pfanne unkontrollierbar mehr oder weniger aus der Operationsöffnung herausgedrückt wird.

Aufgabe der Erfindung ist es deshalb, mindestens in Teilbereichen der Aussenfläche der Pfanne die geforderte Mindestdicke des Knochenzementbettes sicherzustellen. Diese Aufgabe wird dadurch gelöst, dass, entlang Meridianlinien verlaufend, auf dem Metallgitter rippenartige Vorsprünge gleichmässig verteilt sind.

Selbst wenn der Knochenzement an den Vorsprüngen in das Implantat und die Spongiosa so weit eindringt bzw. so weit verdrängt wird, dass das Implantat dort am Knochen "ansteht", bleibt zwischen den Vorsprüngen die geforderte Mindestdicke des Zementbettes erhalten. Gleichzeitig bilden die Mulden zwischen den Vorsprüngen Auffangbecken für den an den Vorsprüngen verdrängten Knochenzement. Schliesslich stellen die Vorsprünge und das zwischen ihnen liegende Knochenzementbett eine Rotationssicherung für die Pfanne gegen unbeabsichtigte Rotation dar.

Als "Muster" für eine ausreichende Teilfläche mit der geforderten Mindestdicke an Knochenzement und für eine optimale und gleichmässige Kraftübertragung hat es sich bewährt, wenn die Vorsprünge zu beiden Seiten eines rippenfreien Bereichs in mittleren Breiten versetzt zueinander angeordnet sind, und/oder wenn der Polbereich gegenüber den zu ihm gerichteten Enden der Vorsprünge versenkt ist.

Im folgenden wird die Erfindung anhand eines Ausführungsbeispiels im Zusammenhang mit der Zeichnung näher erläutert.

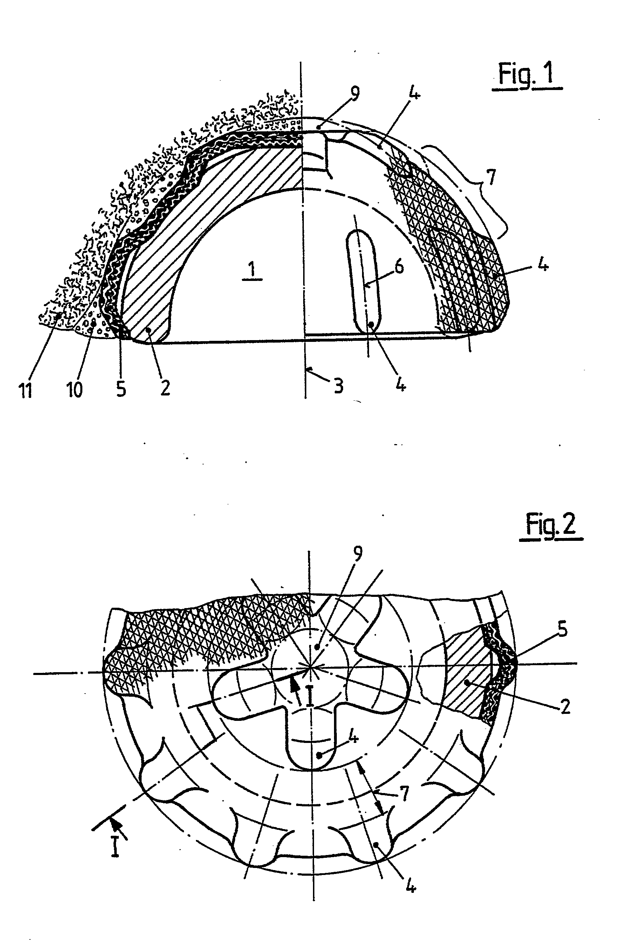

Fig. I ist schematisch und teilweise im Schnitt I-I von Fig. 2 eine Seitenansicht der neuen Endoprothese;

Fig. 2 gibt eine Aufsicht auf den Pfannenpol von aussen in Richtung der Pfannenachse wieder.

Die den nicht gezeigten Gelenkkopf einer Femurkopfprothese aufnehmende halbkugelförmige Pfannenschale I befindet sich in einem Pfannenkörper 2 aus Kunststoff, beispielsweise aus Polyäthylen. Pfannenschale I und Pfannenkörper 2 sind rotationssymmetrisch zur Pfannenachse 3 ausgebildet.

Auf die Aussenfläche des Pfannenkörpers 2 ist als Verstärkungselement, das zugleich zur Verankerung der Pfanne im Beckenknochen dient, ein Metallgitter in Form eines mehrlagigen Drahtnetzes 5 aufgebracht. Pfannenkörper 2 und Drahtnetz 5 sind dadurch fest miteinander verbunden, dass die innerste Lage des Netzes in den Kunststoff eingepresst und daher mindestens teilweise mit ihm gefüllt ist.

Erfindungsgemäss sind auf dem Umfang des Drahtnetzes 5 rippenartige Vorsprünge oder Wulste 4 gleichmässig verteilt, die entlang von Meridianlinien 6 der halbkugelförmigen Aussenfläche der Pfanne verlaufen; sie sind durch mechanische Verformungen des Netzes vor dem Einpressen des Kunststoffkörpers 2 erzeugt.

In "mittleren geographischen Breiten" der Aussenfläche ist ein rippenfreier Bereich 7 vorhanden. Die zu beiden Seiten dieses Bereichs 7 - d.h. zum Aequator und zum Pol hin - aufgeprägten Wulste 4 sind versetzt zueinander angeordnet. Weiterhin ist die Pfanne im Polbereich 9 gegenüber einer "ungestörten" Halbkugel und gegenüber den Enden der diesen Bereich begrenzenden Wulste 4 versenkt.

Auf diese Weise entstehen zwischen den Wulsten 4 relativ grossflächige Bereiche, in denen ein ausreichend tiefes Knochenzementbett I0 zwischen dem Drahtnetz 5 und dem spongiosen Knochen II vorhanden ist.

## Patentansprüche

I. In einem Knochenzementbett zu verankernde Endoprothese für eine Hüftgelenkspfanne, bestehend aus einem inneren Kunststoff-Pfannenkörper, der die eigentliche Pfannenschale aufnimmt, und aus einem mehrlagigen Metallgitter, das auf der im wesentlichen halbkugelförmigen äusseren Oberfläche des Pfannenkörpers fixiert ist, dadurch gekennzeichnet, dass, entlang Meridianlinien (6) verlaufend, auf dem Metallgitter (5) rippenartige Vorsprünge (4) gleichmässig verteilt sind.

2. Endoprothese nach Anspruch I, dadurch gekennzeichnet, dass die Vorsprünge (4) zu beiden Seiten eines in mittleren Breiten befindlichen, rippenfreien Bereichs (7) versetzt zueinander angeordnet sind.

3. Endoprothese nach Anspruch I oder 2, dadurch gekennzeichnet, dass der Polbereich (9) gegenüber den zu ihm gerichteten Enden der Vorsprünge (4) versenkt ist.

4. Endoprothese nach einem der Ansprüche I–3, dadurch gekennzeichnet, dass das Innere der Vorsprünge (4) mindestens teilweise mit dem Material des Pfannenkörpers (2) ausgefüllt sind.

## Claims

1. An endoprosthesis for an acetabulum, the prosthesis being adapted to be anchored in a bone cement bed and comprising an inner plastics cup or socket, the same receiving the actual shell, and a multilayer metal mesh secured to the substantially hemispherical outer surface of the cup or socket, characterised in that projections (4) extending along meridian lines (6) are distributed uniformly over the metal mesh (5).

2. An endoprosthesis according to claim 1, characterised in that the projections (4) are arranged in staggered relationship to one another on both sides of a rib-free zone (7) disposed in central zones.

3. An endoprosthesis according to claim 1 or 2, characterised in that the pole zone (9) is recessed relatively to those ends of the projections (4) which extend towards the latter zone.

4. An endoprosthesis according to any of claims 1 – 3, characterised in that the interior of the projections (4) is filled at least to some extent with the material of the cup or socket (2).

## Revendications

1. Endoprothèse pour une calotte coxofémorale, destinée à être ancrée dans un lit d'ostéociment et se composant d'un corps de cuvette interne en matière plastique, qui reçoit l'enveloppe de la calotte proprement dite, ainsi que d'un tressage métallique à plusieurs couches qui est consigné à demeure sur la surface externe, sensiblement hémisphérique, du corps de la cuvette, caractérisée par le fait que des protubérances (4) du type nervures, s'étendant le long de lignes méridiennes (6), sont uniformément réparties sur le tressage métallique (5).

2. Endoprothèse selon la revendication 1, caractérisée par le fait que les protubérances (4) sont agencées avec décalage mutuel, de part et d'autre d'une région (7) qui est dépourvue de nervures et se trouve dans des largeurs moyennes.

3. Endoprothèse selon la revendication 1 ou 2, caractérisée par le fait que la zone polaire (9) est encaissée par rapport aux extrémités des protubérances (4), qui sont tournées vers cette zone.

4. Endoprothèse selon l'une des revendications 1–3, caractérisée par le fait que l'espace interne des protubérances (4) est comblé, au moins en partie, par le matériau du corps (2) de la cuvette.

Fig. 1

Fig. 2